(19)

Europäisches
Patentamt
European
Patent Office
Office européen
des brevets

(11) **EP 3 276 380 A1**

(12) **EUROPEAN PATENT APPLICATION**
published in accordance with Art. 153(4) EPC

(43) Date of publication:
**31.01.2018 Bulletin 2018/05**

(21) Application number: **16768351.5**

(22) Date of filing: **03.03.2016**

(51) Int Cl.:
**G01T 1/161** $^{(2006.01)}$

(86) International application number:
**PCT/JP2016/056682**

(87) International publication number:
**WO 2016/152448 (29.09.2016 Gazette 2016/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.03.2015 JP 2015060546**

(71) Applicant: **Nihon Medi-Physics Co., Ltd.**
**Tokyo 136-0075 (JP)**

(72) Inventor: **NISHIKAWA, Kazuhiro**
**Tokyo 136-0075 (JP)**

(74) Representative: **TBK**
**Bavariaring 4-6**
**80336 München (DE)**

(54) **IMAGE PROCESSING APPARATUS, IMAGE PROCESSING METHOD, AND PROGRAM**

(57)     An image processing device (1) comprises: an image data acquisition unit (10) for acquiring SPECT image data of a brain; a brain-region ROI definition unit (13) for defining a brain-region ROI in the SPECT image; a striatum ROI definition unit (14) for defining a striatum ROI in the SPECT image; a histogram generation unit (15) for generating a histogram whose horizontal axis represents the class of counts and whose vertical axis represents the frequency of unit region for each class; a threshold determination unit (16) for, based on counts in the SPECT image's background which is the brain-region ROI except the striatum ROI, determining a threshold for distinguishing ventricles and sulci in the SPECT image; a region distinction unit (17) for distinguishing between a region whose number of counts is smaller than or equal to the threshold and a region whose number of counts is larger than the threshold; and an SBR calculation unit (18) for calculating an SBR (Specific Binding Ratio) with the exception of the region whose number of counts is smaller than or equal to the threshold. This allows for using only nuclear medicine image data to eliminate the influence of ventricles and sulci in the calculation of the SBR.

Fig.1

**Description**

TECHNICAL FIELD

The present invention relates to a technique for image processing of a nuclear medicine image of the brain, and particularly to a technique for determining an SBR (specific binding ratio), which is a quantitative index of specific binding of the striatal dopamine transporter.

BACKGROUND ART

The dopamine transporter highly expresses in nerve terminals of nigrostriatal dopamine neurons existing in the brain striatum. This expression level is known to decrease in cases of Parkinson's disease and Lewy body dementia, and therefore the intracerebral distribution of the dopamine transporter is evaluated by PET or SPECT examination using $^{123}$I-ioflupane, $^{18}$F-DOPA, or other radiopharmaceuticals for the diagnosis of these diseases.

Since visual assessment of SPECT images alone cannot prevent variation in assessment due to readers' experiences or the like, it is recommended to add a quantitative index to the diagnosis, and an SBR (specific binding ratio) is used as a quantitative index of specific binding of $^{123}$I-ioflupane in the striatum, where the SBR is determined by (radioactivity due to specific binding)/(radioactivity due to non-specific binding).

In this regard, the volume of the striatum is usually about 11 ml even if it is normal without atrophy and, given the spatial resolution of SPECT images, is known to be affected by the partial volume effect. If a small ROI (region of interest) is defined in the striatum, the partial volume effect causes a decrease in counts in the striatum ROI, which leads to a decrease and change in its quantitative index. The inter-operator reproducibility is also regarded as a problem. The Bolt method has been proposed as a method to cover these shortcomings, in which a thick (44 mm) striatum-centered tomographic image is used and a large striatum-centered ROI is defined (Non-patent document 1).

PRIOR ART DOCUMENT

Non-Patent Document

Non-patent document 1: Tossici-Bolt, L. et al., Quantification of [123I]FP-CIT SPECT brain images: an accurate technique for measurement of the specific binding ratio, Eur J Nucl Med Mol Imaging 2006; 33: 1491-9.

SUMMARY OF THE INVENTION

Problems to be solved by the invention

The brain includes ventricles and sulci where the cerebrospinal fluid (CSF) is present and these do not accept $^{123}$I-ioflupane, so no radiation count can be obtained there. Since in the Bolt method a large striatum-centered ROI is defined and ventricles and sulci are included in the relevant ROI and a background ROI used as a reference region, the SBR is affected. Even when the Bolt method is not used, ventricles and sulci are included around the reference region, and the calculation of the BP (binding potential) and SBR may be affected.

There is also a technique in which a mask exclusive of ventricles and sulci are created by using other imaging results including MRI in order to eliminate the above effect and the mask is superimposed over SPECT images. However, this requires MRI or other imaging facility, data handling for the mask creation process, and complicated processing, which present problems.

In view of the above, a purpose of the invention is to provide a device and method capable of eliminating the influence of ventricles and sulci using only nuclear medicine image data.

Means for solving the problems

An image processing device of the present embodiment comprises: an image data acquisition unit for acquiring nuclear medicine image data of a brain; a brain-region ROI definition unit for defining a brain-region ROI in the nuclear medicine image; a striatum ROI definition unit for defining a striatum ROI containing the striatum in the nuclear medicine image; and a threshold determination unit for determining a threshold for distinguishing ventricles and sulci in the nuclear medicine image based on counts in the nuclear medicine image's background which is the brain-region ROI except the striatum ROI, where a region whose number of counts is smaller than or equal to the threshold and a region whose number of counts is larger than the threshold are distinguished in the nuclear medicine image.

The above use of counts in the background which is the brain-region ROI except the striatum ROI allows for determining an appropriate threshold for distinguishing ventricles and sulci using only nuclear medicine image data, distinguishing a region whose number of counts is smaller than or equal to the threshold (that is the region of ventricles and sulci), and effectively eliminating the influence of ventricles and sulci.

The image processing device of the present embodiment may comprise a histogram generation unit for determining the number of counts for each unit region in the background and generating a histogram whose horizontal axis represents the class of counts and whose vertical axis represents the frequency of unit region for each class, where the threshold determination unit fits a normal distribution to the histogram and determines the threshold based on at least one of the median and standard deviation of the normal distribution. As an example, the threshold may be determined by (the median of the normal distribution) - (the standard deviation of the normal distribution) $\times$ (a predetermined coefficient), where the predetermined coefficient may be 1. An appropriate threshold can be determined with this configuration. If the unit region is each pixel, SPECT image data may be used as it is.

The image processing device of the present embodiment may comprise a histogram generation unit for determining the number of counts for each unit region in the background and generating a histogram whose horizontal axis represents the class of counts and whose vertical axis represents the frequency of unit region for each class, where the threshold determination unit determines as the threshold the number of counts for a class which corresponds to a frequency that is smaller than a maximum frequency multiplied by a predetermined coefficient, the class being smaller than the class that gives the maximum frequency. The threshold can be easily determined with this configuration.

The image processing device of the present embodiment may comprise a calculation unit for calculating an index of specific binding of a radiopharmaceutical using data of a region whose number of counts is larger than the threshold. In this regard, the BP (binding potential) and the SBR (specific binding ratio) can be used as the index of specific binding of a radiopharmaceutical.

An image processing method of the present embodiment is for nuclear medicine image data of a brain, and comprises the steps of: an image processing device acquiring nuclear medicine image data of a brain; the image processing device defining a brain-region ROI in the nuclear medicine image; the image processing device defining a striatum ROI containing the striatum in the nuclear medicine image; the image processing device determining a threshold for distinguishing ventricles and sulci in the nuclear medicine image based on counts in the nuclear medicine image's background which is the brain-region ROI except the striatum ROI; and distinguishing in the nuclear medicine image a region whose number of counts is smaller than or equal to the threshold and a region whose number of counts is larger than the threshold. The configurations described with the above-described image processing device may be applied to the method of specifically determining the threshold in the image processing method of the invention. An index of specific binding of a radiopharmaceutical may be calculated by using data of the region whose number of counts is larger than the threshold, the region distinguished in the image processing method of the invention. In a preferred mode, the index of specific binding of a radiopharmaceutical can be the BP (binding potential) and the SBR (specific binding ratio).

A program of the present invention causes a computer to execute the above-described image processing method. A recording medium on which a program of the present invention is recorded is a recording medium on which the program causing a computer to execute the above-described image processing method is recorded.

BRIEF DESCRIPTION OF THE DRAWINGS

Figure 1 shows the configuration of an image processing device of a first embodiment;
Figure 2 shows an example of a striatum ROI containing the striatum;
Figure 3A shows an example of a histogram of counts in a background;
Figure 3B shows an example in which a normal distribution is fitted to the histogram;
Figure 4 shows how to determine a threshold in the first embodiment;
Figure 5 shows an example of an image processing program;
Figure 6 shows an operation of the image processing device of the first embodiment;
Figure 7A shows an example of a histogram of counts in a background;
Figure 7B shows how to determine a threshold in a second embodiment;
Figure 8 shows a histogram created from a background and its normal distribution;
Figure 9A shows a result of a comparison between an SBR determined without eliminating the influence of ventricles and sulci and the true value of the SBR; and
Figure 9B shows a result of a comparison between an SBR determined by the method of the invention and the true value of the SBR.

MODES OF EMBODYING THE INVENTION

Now, the image processing device of embodiments of the invention will be described with reference to the drawings. The examples described below are only exemplary of preferred modes and are not intended to limit the subject matter of the invention.

(First embodiment)

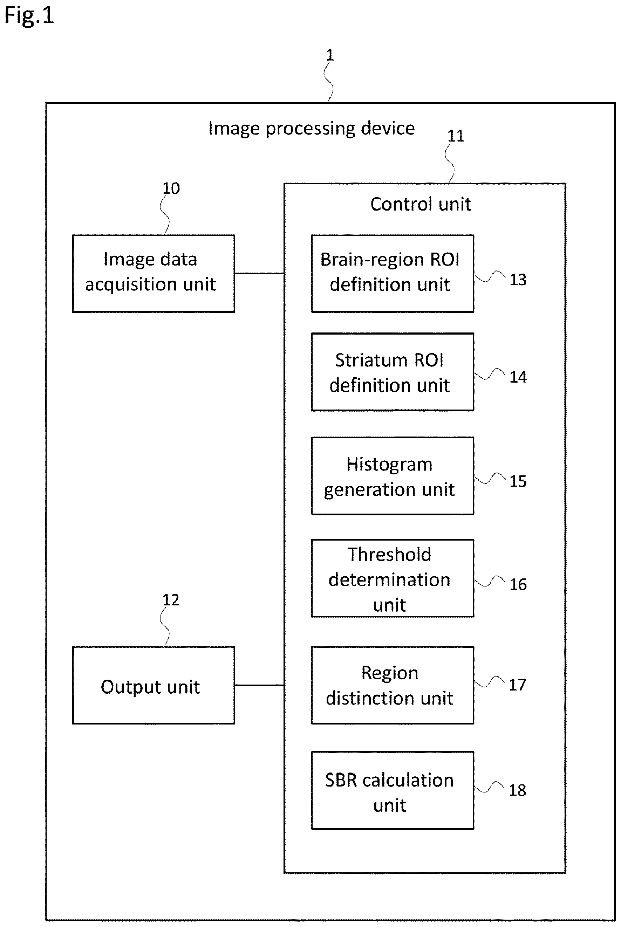

Figure 1 shows the configuration of an image processing device 1 of a first embodiment of the invention. The image processing device 1 has: an image data acquisition unit 10 for acquiring brain SPECT image data; a control unit 11 for processing the SPECT image data to distinguish the region of ventricles and sulci and calculating an SBR which is a quantitative index of specific binding in the striatum; and an output unit 12 for outputting the calculation result.

SPECT image data used in this example is obtained by SPECT examination using $^{123}$I-ioflupane. Radiation emitted from $^{123}$I-ioflupane is detected by using a dedicated gamma camera, and the image data is made from the distribution. Each pixel has the number of radiation events, i.e. counts.

In a preferred mode, the control unit 11 has a brain-region ROI definition unit 13, a striatum ROI definition unit 14, a histogram generation unit 15, a threshold determination unit 16, a region distinction unit 17, and an SBR calculation unit 18.

The brain-region ROI definition unit 13 locates and defines in a SPECT image a region where a brain region exists. Methods of defining the brain-region ROI include, for example, a method in which a user uses a mouse, a stylus pen, or the like to manually enclose the brain parenchyma to define the region, and a method in which the region is defined based on an image contour determined by a threshold method. When the region is defined based on an image contour, the brain-region ROI may be the image contour itself, or may be a region defined by pixels a few pixels (actually about 20 mm) inside the contour. There is also a method in which the brain-region ROI is defined by a circle, an ellipse, or other standard shape figures containing the brain parenchyma. In this case, it is desirable for the brain-region ROI not to extend off the brain parenchyma.

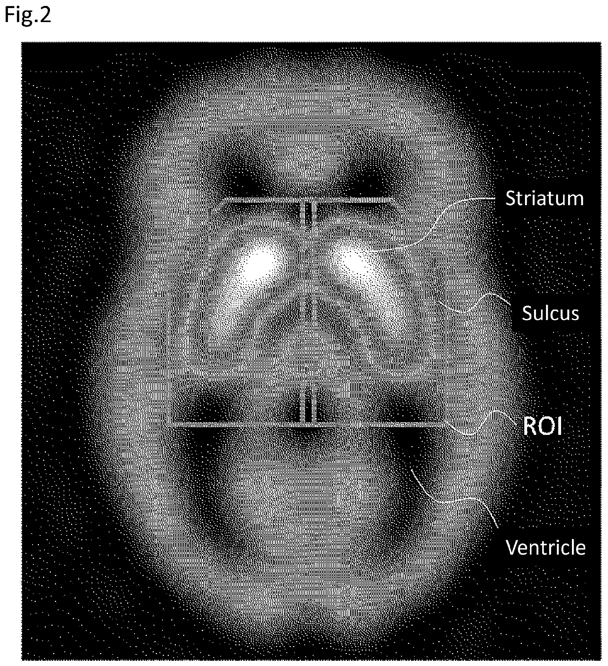

As shown in Figure 2, the striatum ROI definition unit 14 defines as the striatum ROI a predetermined region containing the striatum in a brain SPECT image. The region of the brain-region ROI except the striatum region in SPECT image data is herein called the "background (B.G.)." The SBR calculation unit 18 is of course illustrative, and may be any that has the function to calculate an index of specific binding of a radiopharmaceutical. The term "SBR calculation unit" is only intended to show a representative calculation to facilitate understanding of the summary of the invention.

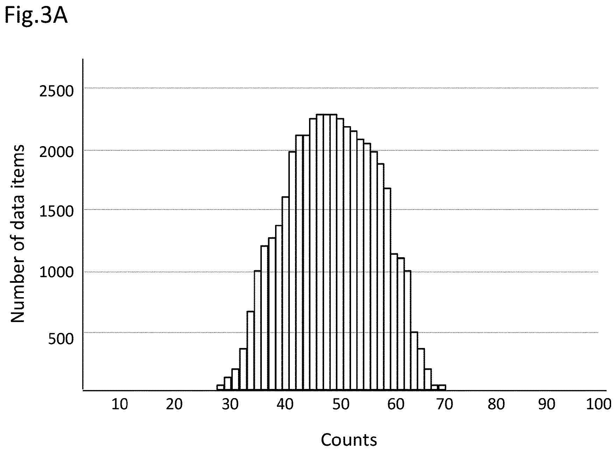

The histogram generation unit 15 generates a histogram of counts in a background. Figure 3A shows an example of a histogram generated by the histogram generation unit 15. The horizontal axis of the histogram represents the class of counts, and the vertical axis of the histogram represents the number of pixels having counts for the relevant class. Though the frequency is determined in terms of pixels in the example, the frequency may be determined for each class in terms of certain regions including, for example, $2 \times 2$ pixels instead.

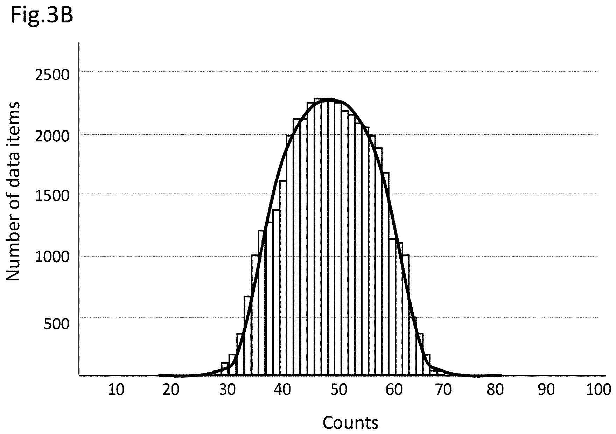

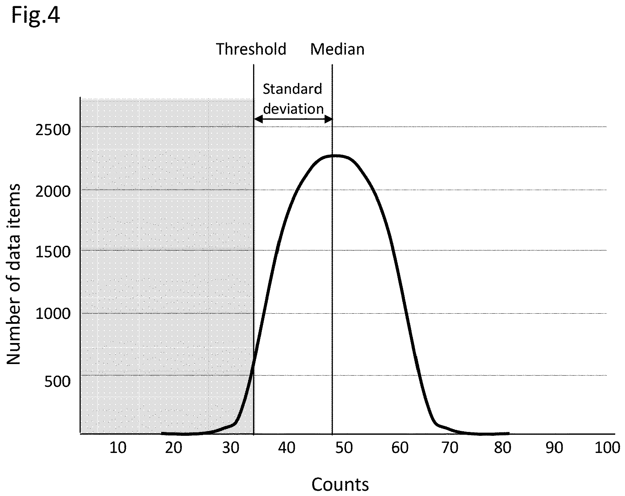

The threshold determination unit 16 has a function to determine a threshold based on a histogram. In a preferred mode, the threshold determination unit 16 first fits a normal distribution to a histogram. Figure 3B shows an example in which a normal distribution is fitted to the histogram shown in Figure 3A. The threshold determination unit 16 then subtracts the standard deviation of the normal distribution from the median of the normal distribution to determine the threshold as shown in Figure 4. This threshold is intended to regard regions whose number of counts is smaller than or equal to the threshold as ventricles or sulci. The region distinction unit 17 identifies regions whose number of counts is smaller than or equal to the threshold as regions of ventricles or sulci, and regions whose number of counts is larger than the threshold as regions other than those.

The SBR calculation unit 18 eliminates data of the regions whose number of counts is smaller than or equal to the above-described threshold (i.e. regions of ventricles and sulci) distinguished by the region distinction unit 17, and calculates the SBR. The calculation of the SBR is the same as the conventional method except for the elimination of data of parts determined to be ventricles and sulci by using the above-described threshold. Specifically, the SBR is determined by the following equation (1).

$$SBR = [(\text{ROI average counts}) - (\text{B.G. average counts})]/(\text{B.G. average counts}) \qquad (1)$$

The image processing device 1 is composed of a computer comprising: a CPU; RAM; ROM; a display; a keyboard; a mouse; a communications interface; and the like. The computer calculates the SBR based on SPECT image data in a manner that a program for calculating the SBR is stored in ROM in advance and the CPU reads the program from ROM and runs it.

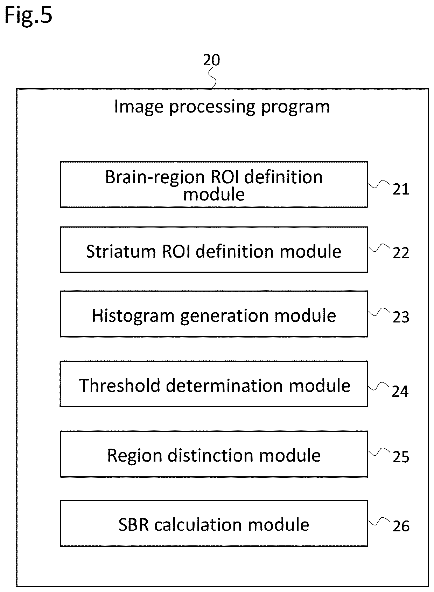

Figure 5 shows an example of an image processing program 20 stored in ROM. The image processing program 20 has a brain-region ROI definition module 21, a striatum ROI definition module 22, a histogram generation module 23, a threshold determination module 24, a region distinction module 25, and an SBR calculation module 26. Each of the modules 21 through 26 causes the computer to execute the program 20 to realize each function of the control unit 11 described above. While the image processing program 20 stored in ROM has been described here, the image processing described in the present embodiment may be realized by recording the image processing program on a computer-readable recording medium and causing a computer to read the recording medium and run the program. The image processing program may also be downloaded to a computer via a communications line.

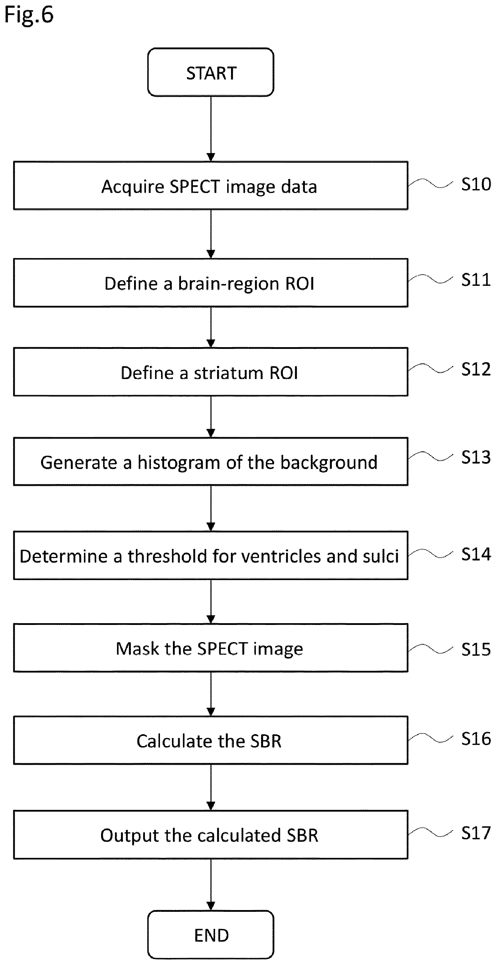

Figure 6 is a flowchart showing an operation of the image processing device 1. The image processing device 1 first acquires SPECT image data (S10). The image processing device 1 may acquire the SPECT image data directly from a SPECT device, or from a database in which the SPECT image data is stored.

The image processing device 1 then defines a brain-region ROI (S11) and a striatum ROI in a region containing the striatum (S12) in the SPECT image, and generates a histogram of counts in a region of the brain-region ROI except the striatum ROI (background) (S13). The image processing device 1 subsequently fits a normal distribution to the histogram, and determines a threshold by subtracting the standard deviation from the median (S14).

The image processing device 1 then distinguishes regions in the SPECT image whose number of counts is smaller than or equal to the threshold, and generates a mask for excluding the regions (S15). That is, the regions whose number of counts is smaller than or equal to the threshold are not included in the subsequent calculation. The image processing device 1 uses the masked data to determine the average counts for the striatum ROI and the average counts for the background, and calculates the SBR using the equation (1) described above (S16). The image processing device 1 outputs the calculated SBR (S17).

There has been described the image processing device 1 and image processing method of the first embodiment of the invention. The image processing device 1 of the first embodiment uses counts in the background to determine the threshold, and can therefore appropriately determine the threshold for distinguishing ventricles and sulci using only SPECT image data. This threshold can be used to eliminate the influence of ventricles and sulci to determine the SBR.

While in the present embodiment the threshold is determined by (the median) - (the standard deviation), the threshold may be determined by subtracting the standard deviation multiplied by a predetermined coefficient from the median. It is preferable that this predetermined coefficient is determined so that the SBR comes closest to the true value determined by a predetermined method.

(Second embodiment)

An image processing device of a second embodiment of the invention will now be described. The configuration of the image processing device of the second embodiment is basically the same as the image processing device 1 of the first embodiment, but is different therefrom in that it determines the threshold for distinguishing ventricles and sulci in a different manner.

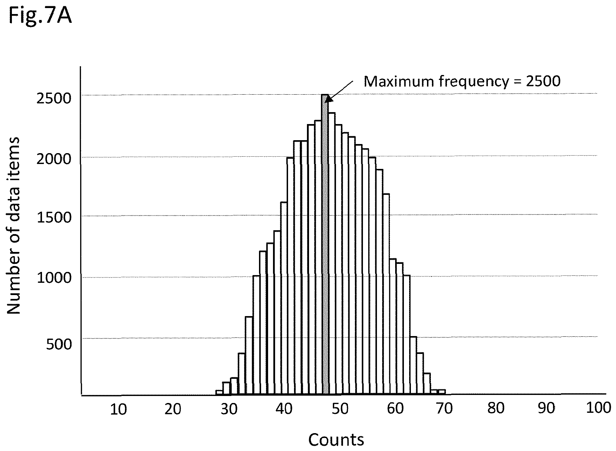

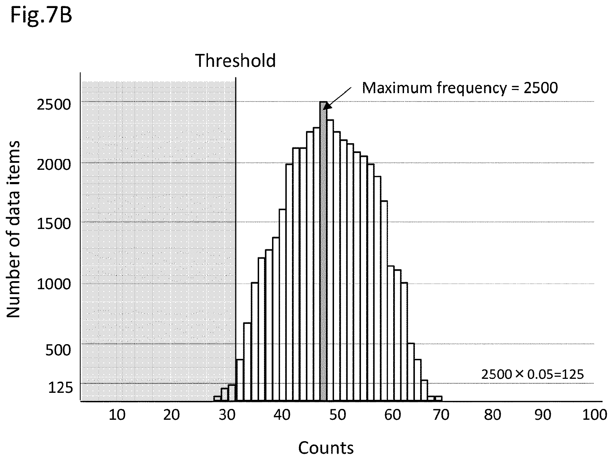

Figures 7A and 7B illustrate how to determine the threshold in the second embodiment. As shown in Figure 7A, the maximum frequency is determined in the histogram in the second embodiment. This maximum frequency is then multiplied by a predetermined coefficient, which is 0.05 in the present embodiment. The maximum frequency in the example shown in Figure 7A is 2500, which is multiplied by 0.05, resulting in 125. The threshold determination unit 16 determines as the threshold the number of counts for a class whose frequency is smaller than or equal to 125. In this regard, a class whose frequency is smaller than or equal to 125 also exists among classes with a large number of counts, but the threshold is chosen from classes with a small number of counts. That is, a class is chosen that is smaller than the class that gives the maximum frequency. More specifically, classes are checked in descending order from the class with the maximum frequency, and the number of counts for the class that first falls below 125 is determined to be the threshold.

The image processing device of the second embodiment also has the advantage of being able to distinguish regions of ventricles and sulci using only SPECT image data and determining the SBR exclusive of the influence of ventricles and sulci. In addition, since the image processing device of the second embodiment does not have to fit a normal distribution, its process is simple.

Examples

Shown below is the fact that the method of the invention in which the SBR is calculated by using only SPECT image data can provide a calculation close to the true SBR.

(True value of SBR)

A calculation of SBR determined by creating a brain parenchyma mask from an MR image shall be the true value to be compared with the method of the invention. The true value is determined according to the following procedure:

1. A SPECT image is aligned to an MR image;
2. SPM (statistical parametric mapping) is used to extract gray matter and white matter from the MR image;
3. Gray matter and white matter are added up and converted to a binary representation with a threshold of 50%;
4. The binary-converted data is set to be a mask; and
5. Only masked SPECT image data is calculated to determine the SBR.

(SBR without mask)

In order to examine how ventricles and sulci affect the SBR, striatum ROI (and background) data defined in a SPECT image was used to directly determine the SBR without using the masked image described above. The determined SBR is compared with the true value. Each ROI was defined by using "DaTView" in "Nou Toukei Kaiseki Package" (Brain statistical analysis package) contained in AZE VirtualPlace HAYABUSA (made by AZE, Ltd., Medical device approval number: 22000BZX00379000).

(SBR determined by the invention)

The SBR is determined by the method described in the above first embodiment.

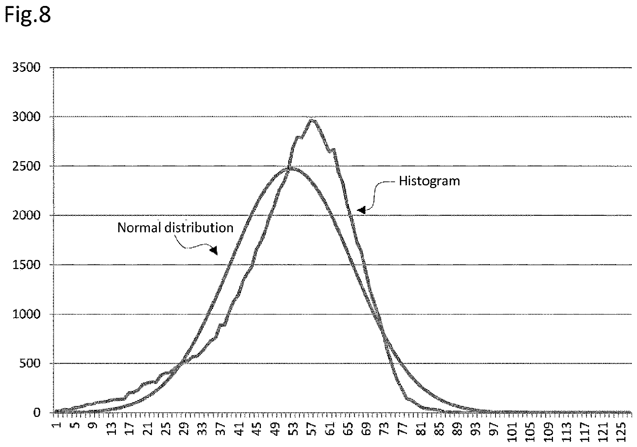

Figure 8 shows a histogram of counts in a background determined by the method described in the first embodiment for one of the used cases. When this histogram is fitted with a normal distribution, the median is 51.65 and the standard deviation is 13.52. The standard deviation of the normal distribution is subtracted from the median of the normal distribution to determine the threshold of 38.03. The SBR is calculated excluding regions whose number of counts is smaller than or equal to this threshold. In this example, the above-described process was executed for each used case, and their own thresholds were determined.

(Comparison result)

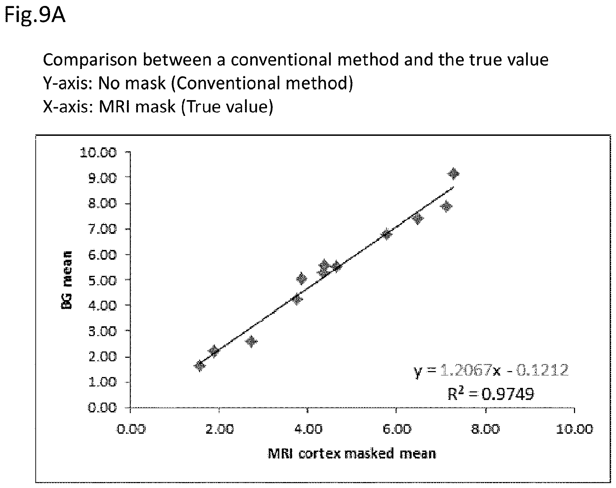

Figure 9A is a graph showing a comparison between an SBR determined by using striatum ROI (and background) data defined in a SPECT image without using a mask image and the true value of the SBR. The vertical axis of the graph represents the SBR determined by using striatum ROI (and background) data defined in a SPECT image without using a mask image, and the horizontal axis represents the true value of the SBR. As shown in Figure 9A, a strong correlation was seen between the SBR determined without using a mask and the true value, but the SBR is overvalued by approximately 20%.

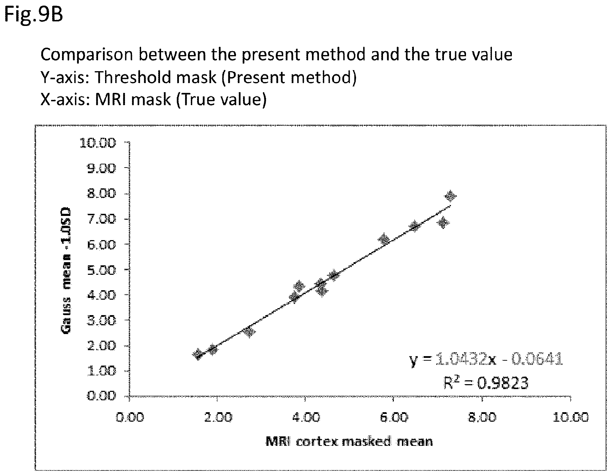

Figure 9B is a graph showing a comparison between an SBR determined by the method of the invention and the true value of the SBR. As shown in Figure 9B, the slope of the obtained line is almost 1, which indicates that a result extremely close to the true value can be obtained by using the method of the invention.

Industrial applicability

The invention allows for distinguishing regions of ventricles and sulci using only nuclear medicine image data, and is useful for SPECT image processing.

DESCRIPTION OF THE SYMBOLS

1: Image processing device
10: Image data acquisition unit
11: Control unit
12: Output unit
13: Brain-region ROI definition unit
14: Striatum ROI definition unit
15: Histogram generation unit
16: Threshold determination unit

17: Region distinction unit
18: SBR calculation unit
20: Image processing program
21: Brain-region ROI definition module
22: Striatum ROI definition module
23: Histogram generation module
24: Threshold determination module
25: Region distinction module
26: SBR calculation module

**Claims**

1. An image processing device comprising:

    an image data acquisition unit for acquiring nuclear medicine image data of a brain;
    a brain-region ROI definition unit for defining a brain-region ROI in the nuclear medicine image;
    a striatum ROI definition unit for defining a striatum ROI containing the striatum in the nuclear medicine image; and
    a threshold determination unit for determining a threshold for distinguishing ventricles and sulci in the nuclear medicine image based on counts in the nuclear medicine image's background which is the brain-region ROI except the striatum ROI, wherein a region whose number of counts is smaller than or equal to the threshold and a region whose number of counts is larger than the threshold are distinguished in the nuclear medicine image.

2. The image processing device according to claim 1, comprising
   a histogram generation unit for determining the number of counts for each unit region in the background and generating a histogram whose horizontal axis represents the class of counts and whose vertical axis represents the frequency of unit region for each class,
   wherein the threshold determination unit fits a normal distribution to the histogram and determines the threshold based on at least one of the median and standard deviation of the normal distribution.

3. The image processing device according to claim 2, wherein the threshold determination unit determines the threshold by (the median of the normal distribution) - (the standard deviation of the normal distribution) $\times$ (a predetermined coefficient).

4. The image processing device according to claim 3, wherein the predetermined coefficient is 1.

5. The image processing device according to claim 1, comprising
   a histogram generation unit for determining the number of counts for each unit region in the background and generating a histogram whose horizontal axis represents the class of counts and whose vertical axis represents the frequency of unit region for each class,
   wherein the threshold determination unit determines as the threshold the number of counts for a class which corresponds to a frequency that is smaller than a maximum frequency multiplied by a predetermined coefficient, the class being smaller than the class that gives the maximum frequency.

6. The image processing device according to any of claims 1 to 5, comprising a calculation unit for calculating an index of specific binding of a radiopharmaceutical using data of a region whose number of counts is larger than the threshold.

7. An image processing method for nuclear medicine image data of a brain, comprising the steps of:

    an image processing device acquiring nuclear medicine image data of a brain;
    the image processing device defining a brain-region ROI in the nuclear medicine image;
    the image processing device defining a striatum ROI containing the striatum in the nuclear medicine image;
    the image processing device determining a threshold for distinguishing ventricles and sulci in the nuclear medicine image based on counts in the nuclear medicine image's background which is the brain-region ROI except the striatum ROI; and
    the image processing device distinguishing in the nuclear medicine image a region whose number of counts is smaller than or equal to the threshold and a region whose number of counts is larger than the threshold.

8. The image processing method according to claim 7, comprising the step of
the image processing device determining the number of counts for each unit region in the background and generating a histogram whose horizontal axis represents the class of counts and whose vertical axis represents the frequency of unit region for each class,
wherein the step of determining the threshold involves fitting a normal distribution to the histogram and determining the threshold based on at least one of the median and standard deviation of the normal distribution.

9. The image processing method according to claim 8, wherein the step of determining the threshold involves determining the threshold by (the median of the normal distribution) - (the standard deviation of the normal distribution) × (a predetermined coefficient).

10. The image processing method according to claim 9, wherein the predetermined coefficient is 1.

11. The image processing method according to claim 7, comprising the step of the image processing device determining the number of counts for each unit region in the background and generating a histogram whose horizontal axis represents the class of counts and whose vertical axis represents the frequency of unit region for each class, wherein the step of determining the threshold involves determining as the threshold the number of counts for a class which corresponds to a frequency that is smaller than a maximum frequency multiplied by a predetermined coefficient, the class being smaller than the class that gives the maximum frequency.

12. The image processing method according to any of claims 7 to 11, comprising the step of the image processing device calculating an index of specific binding of a radiopharmaceutical using data of a region whose number of counts is larger than the threshold.

13. A program for image processing of nuclear medicine image data of a brain, the program causing a computer to execute the steps of:

   acquiring nuclear medicine image data of a brain;
   defining a brain-region ROI in the nuclear medicine image;
   defining a striatum ROI containing the striatum in the nuclear medicine image;
   determining a threshold for distinguishing ventricles and sulci in the nuclear medicine image based on counts in the nuclear medicine image's background which is the brain-region ROI except the striatum ROI; and
   distinguishing in the nuclear medicine image a region whose number of counts is smaller than or equal to the threshold and a region whose number of counts is larger than the threshold.

14. The program according to claim 13, causing a computer to execute the step of determining the number of counts for each unit region in the background and generating a histogram whose horizontal axis represents the class of counts and whose vertical axis represents the frequency of unit region for each class,
wherein the step of determining the threshold involves fitting a normal distribution to the histogram and determining the threshold based on at least one of the median and standard deviation of the normal distribution.

15. The program according to claim 14, wherein the step of determining the threshold involves determining the threshold by (the median of the normal distribution) - (the standard deviation of the normal distribution) × (a predetermined coefficient).

16. The program according to claim 15, wherein the predetermined coefficient is 1.

17. The program according to claim 13, causing a computer to execute the step of determining the number of counts for each unit region in the background and generating a histogram whose horizontal axis represents the class of counts and whose vertical axis represents the frequency of unit region for each class,
wherein the step of determining the threshold involves determining as the threshold the number of counts for a class which corresponds to a frequency that is smaller than a maximum frequency multiplied by a predetermined coefficient, the class being smaller than the class that gives the maximum frequency.

18. The program according to any of claims 13 to 17, causing a computer to execute the step of calculating an index of specific binding of a radiopharmaceutical using data of a region whose number of counts is larger than the threshold.

## Fig.1

**1** Image processing device

- **10** Image data acquisition unit
- **11** Control unit
  - **13** Brain-region ROI definition unit
  - **14** Striatum ROI definition unit
  - **15** Histogram generation unit
  - **16** Threshold determination unit
  - **17** Region distinction unit
  - **18** SBR calculation unit
- **12** Output unit

Fig.2

Fig.3A

Fig.3B

Fig.4

Fig.5

20

**Image processing program**

| Brain-region ROI definition module | 21 |

| Striatum ROI definition module | 22 |

| Histogram generation module | 23 |

| Threshold determination module | 24 |

| Region distinction module | 25 |

| SBR calculation module | 26 |

Fig.6

```
                    ┌─────────────┐
                    │    START    │
                    └──────┬──────┘
                           │
                           ▼
         ┌──────────────────────────────────┐
         │      Acquire SPECT image data     │ ～ S10
         └──────────────────┬───────────────┘
                           │
                           ▼
         ┌──────────────────────────────────┐
         │       Define a brain-region ROI    │ ～ S11
         └──────────────────┬───────────────┘
                           │
                           ▼
         ┌──────────────────────────────────┐
         │         Define a striatum ROI      │ ～ S12
         └──────────────────┬───────────────┘
                           │
                           ▼
         ┌──────────────────────────────────┐
         │  Generate a histogram of the background │ ～ S13
         └──────────────────┬───────────────┘
                           │
                           ▼
         ┌──────────────────────────────────┐
         │ Determine a threshold for ventricles and sulci │ ～ S14
         └──────────────────┬───────────────┘
                           │
                           ▼
         ┌──────────────────────────────────┐
         │         Mask the SPECT image       │ ～ S15
         └──────────────────┬───────────────┘
                           │
                           ▼
         ┌──────────────────────────────────┐
         │          Calculate the SBR         │ ～ S16
         └──────────────────┬───────────────┘
                           │
                           ▼
         ┌──────────────────────────────────┐
         │       Output the calculated SBR    │ ～ S17
         └──────────────────┬───────────────┘
                           │
                           ▼
                    ┌─────────────┐
                    │     END     │
                    └─────────────┘
```

## Fig.7A

## Fig.7B

Fig.8

# Fig.9A

Comparison between a conventional method and the true value
Y-axis: No mask (Conventional method)
X-axis: MRI mask (True value)

# Fig.9B

Comparison between the present method and the true value
Y-axis: Threshold mask (Present method)
X-axis: MRI mask (True value)

$y = 1.0432x - 0.0641$
$R^2 = 0.9823$

**EP 3 276 380 A1**

## INTERNATIONAL SEARCH REPORT

| | International application No. |
|---|---|
| | PCT/JP2016/056682 |

**A. CLASSIFICATION OF SUBJECT MATTER**
*G01T1/161(2006.01)i*

According to International Patent Classification (IPC) or to both national classification and IPC

**B. FIELDS SEARCHED**

Minimum documentation searched (classification system followed by classification symbols)
G01T1/161

Documentation searched other than minimum documentation to the extent that such documents are included in the fields searched
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2011-502130 A (Alseres Pharmaceuticals, Inc.), 20 January 2011 (20.01.2011), entire text; all drawings & WO 2009/058754 A1 & CA 2700468 A1 | 1–18 |

☐ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10 May 2016 (10.05.16) | 24 May 2016 (24.05.16) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TOSSICI-BOLT, L. et al.** Quantification of [123I]FP-CIT SPECT brain images: an accurate technique for measurement of the specific binding ratio. *Eur J Nucl Med Mol Imaging,* 2006, vol. 33, 1491-9